# EUROPÄISCHE PATENTSCHRIFT

(11) **EP 3 945 085 B1**
(45) Veröffentlichungstag und Bekanntmachung des Hinweises auf die Patenterteilung: **03.01.2024**
(21) Anmeldenummer: 21184642.3
(22) Anmeldetag: 09.07.2021
(51) Int. Cl.: C07C 45/50, C07C 47/02

(54) **VERFAHREN ZUR HERSTELLUNG VON ALDEHYDEN UND ABTRENNUNG DES KATALYSATORSYSTEMS MITTELS MEMBRANTRENNUNG**
METHOD FOR THE PREPARATION OF ALDEHYDES AND SEPARATION OF THE CATALYST SYSTEM BY MEANS OF MEMBRANE SEPARATION
PROCÉDÉ DE FABRICATION D'ALDÉHYDES ET DE SÉPARATION DU SYSTÈME CATALYTIQUE AU MOYEN DE LA SÉPARATION DE MEMBRANE

(30) Priorität: 30.07.2020 EP 20188523
(43) Veröffentlichungstag der Anmeldung: 02.02.2022
(73) Patentinhaber: Evonik Oxeno GmbH & Co. KG, 45772 Marl (DE)
(72) Erfinder: Brächer, Alexander, 45721 Haltern am See (DE); Knossalla, Johannes, 46514 Gahlen (DE); Fridag, Dirk, 45721 Haltern am See (DE); Franke, Robert, 45772 Marl (DE); Gluth, Frederik, 45472 Mülheim an der Ruhr (DE); Schäpertöns, Marc, 45657 Recklinghausen (DE); Kubis, Christoph, 18211 Nienhagen (DE); Sale, Anna Chiara, 45657 Recklinghausen (DE); Kucmierczyk, Peter, 44628 Herne (DE); Markovic, Ana, 45721 Haltern am See (DE)
(74) Vertreter: Evonik Patent Association

(56) Entgegenhaltungen:
- WO-A1-2007/036424
- WO-A1-2010/097376

## Beschreibung

Die vorliegende Erfindung betrifft ein Verfahren zur Herstellung von Aldehyden aus C2-bis C20-Olefinen mit einer nachfolgenden Membrantrennung zur Abtrennung des homogen gelösten Katalysatorsystems, wobei vor der Membrantrennung ein Gasaustausch durchgeführt wird, mit dem der Partialdruckanteilanteil von Kohlenmonoxid oder Wasserstoff erhöht wird, um den Katalysatorrückhalt der Membran zu verbessern.

Hydroformylierungsverfahren zur Herstellung von Aldehyden sind dem Fachmann seit langer Zeit bekannt. Dabei werden Olefine mit Synthesegas, einer Mischung aus Kohlenmonoxid (CO) und Wasserstoff (H₂), in Gegenwart eines Katalysatorsystems zu den entsprechenden Aldehyden umgesetzt. Die Hydroformylierung ist ein großtechnisch genutztes Verfahren, welches in Anlagen betrieben wird, die hunderte Kilotonnen (kt) pro Jahr Aldehyd produziert werden können. Dabei typischerweise eingesetzte Katalysatorsysteme sind homogen gelöste Katalysatorsysteme und umfassen Übergangsmetallkomplexe aus zumeist Cobalt oder Rhodium als Metall und phosphorhaltigen Liganden.

Um solche Hydroformylierungsverfahren im Maßstab von hunderten kt wirtschaftlich betreiben zu können kommt es auf verschiedene Faktoren an. Ein wichtiger Faktor ist das Metall des homogen gelösten Katalysatorsystems, da Rhodium und Cobalt bzw. deren als Katalysatorvorstufe eingesetzten Verbindungen relativ hochpreisige Rohstoffe sind. Ein Kernziel beim Betrieb großtechnischer Hydroformylierungsverfahren ist deshalb die Minimierung von Katalysatorverlusten während des Betriebs. Eine bekannte Maßnahme ist die Abtrennung des homogen gelösten Katalysatorsystem aus dem Reaktionsaustrag und Rückführung des Katalysatorsystems in die Reaktionszone. Das kann beispielsweise mittels bekannter Membrantrennverfahren erfolgen (siehe z. B. WO 2007/036424 A1 und WO 2010/097376 A1).

Die bekannten Membrantrennverfahren werden dabei so durchgeführt, dass entweder der mit Synthesegas beaufschlagte Reaktionsaustrag der Membrantrennung zugeführt wird oder Reaktionsaustrag zunächst einer thermischen Trennung (Destillation, Verdampfung im Dünnschichtverdampfer (DSV)) unterzogen wird, wobei der Reaktionsaustrag zunächst entspannt werden muss, und anschließend die Membrantrennung, optional unter Beaufschlagung eines Inertgases wie Stickstoff, erfolgt.

Bei oder vor den bekannten Abtrennverfahren können jedoch weiterhin Katalysatorverluste auftreten, sei es durch Anhaftungen von Clustern in Teilen der Anlage, durch den Ausfall von unlöslichen Übergangsmetallverbindungen in der Anlage oder auch eine unzureichende Abtrennung des Katalysatorsystems aus dem Reaktoraustrag.

Die Aufgabe der vorliegenden Erfindung war deshalb die Bereitstellung eines Verfahrens zur Herstellung von Aldehyden, bei dem die Katalysatorverluste bei der Abtrennung des homogen gelösten Katalysatorsystem mittels Membrantrennung gegenüber bekannten Verfahren verringert werden.

Es hat sich überraschend herausgestellt, dass diese Aufgabe dadurch gelöst werden kann, dass nach der eigentlichen Reaktion, aber vor der Membrantrennung, ein Gasaustausch durchgeführt wird. Dabei wird das Synthesegas, also eine Mischung, die hauptsächlich Kohlenmonoxid und Wasserstoff umfasst, zumindest partiell gegen Kohlenmonoxid oder Wasserstoff ausgetauscht, wodurch der Partialdruckanteil von Kohlenmonoxid oder Wasserstoff erhöht wird. Der produkthaltige Reaktionsaustrag oder der Austrag aus einer vor der Membrantrennung angeordneten thermischen Trennung wird somit im Vergleich zur Reaktion mit einem Gas anderer Zusammensetzung bzw. mit einem Gasgemisch anderer Mischung beaufschlagt, was in der sich anschließenden Membrantrennung zur Erhöhung des Rückhalts und damit zu einer besseren Abtrennung des homogen gelösten Katalysatorsystems führt.

Erfindungsgemäß wird die Aufgabe demnach durch ein Verfahren zur Herstellung von Aldehyden gelöst, wobei das Verfahren zumindest
eine Hydroformylierung durch Umsetzung von C2- bis C20-Olefinen, vorzugsweise C2- bis C17-Olefinen, weiterhin bevorzugt C3 bis C14-Olefinen und ganz besonders bevorzugt C6- bis C14-Olefinen, mit Synthesegas in Gegenwart eines homogen gelösten Katalysatorsystems, welches mindestens Co oder Rh und vorzugsweise einen phosphorhaltigen Liganden umfasst, in mindestens einer Reaktionszone unter Erhalt eines flüssigen produkthaltigen Reaktionsaustrags, wobei bei der Hydroformylierung der Partialdruckanteil von CO oder H2 maximal 75% des Gesamtgasdrucks, vorzugsweise maximal 70% des Gesamtgasdrucks, besonders bevorzugt maximal 65% des Gesamtgasdrucks ausmacht, wobei der Gesamtgasdruck die Summe der vorliegenden Drücke aller vorhandenen gasförmigen Stoffe ist, und
eine Membrantrennung zur Abtrennung des homogen gelösten Katalysatorsystems umfasst, dadurch gekennzeichnet, dass
vor der Membrantrennung ein Gasaustausch mit CO oder H₂ durchgeführt wird, wodurch der Partialdruckanteil von CO oder H₂ mehr als 80% des Gesamtgasdrucks, vorzugsweise mehr als 85% des Gesamtgasdrucks, besonders bevorzugt mehr als 90% des Gesamtgasdrucks ausmacht.

Der erste Teil des erfindungsgemäßen Verfahrens ist die Hydroformylierung der C2-C20-Olefine, vorzugsweise C2- bis C17-Olefine, weiterhin bevorzugt C3 bis C14-Olefine und ganz besonders bevorzugt C6- bis C14-Olefine. Bei dem Verfahren können grundsätzlich die reinen Olefine eingesetzt werden. Eingesetzt werden vorzugsweise jedoch Kohlenwasserstoffströme, die die entsprechenden Olefine enthalten. Die Menge an Olefinen in den Kohlenwasserstoffströmen sollte verständlicherweise ausreichend hoch sein, um eine Hydroformylierung wirtschaftlich betreiben zu können. Bevorzugt enthalten die olefinhaltigen Einsatzgemische praktisch keine weiteren ungesättigten Verbindungen und mehrfach ungesättigte Verbindungen wie Diene oder Acetylenderivate.

Die beim erfindungsgemäßen Verfahren einsetzbaren Kohlenwasserstoffströme können Olefine mit end- und/oder innenständigen C-C-Doppelbindungen umfassen. Die genannten Kohlenwasserstoffströme können außerdem Olefine mit gleicher oder unterschiedlicher Kohlenstoffatomzahl umfassen. Als Olefine eignen sich insbesondere Ethen, Propen, 1- oder 2-Buten oder Mischungen davon, Isobuten, 1- oder 2-Penten oder Mischungen davon, Isopentene, 1-, 2- oder 3-Hexen, 1-Hepten, lineare Heptene mit innenständiger Doppelbindung (2-Hepten, 3-Hepten usw.), Mischungen linearer Heptene, 2- oder 3-Methyl-1-hexen, 1-Octen, lineare Octene mit innenständiger Doppelbindung, Mischungen linearer Octene, 2- oder 3-Methylhepten, 1-Nonen, lineare Nonene mit innenständiger Doppelbindung, Mischungen linearer Nonene, 2-, 3- oder 4-Methyloctene, 1-, 2-, 3-, 4- oder 5-Decen, 2-Ethyl-1-octen, 1-Dodecen, lineare Dodecene mit innenständiger Doppelbindung, Mischungen linearer Dodecene, 1-Tetradecen, lineare Tetradecene mit innenständiger Doppelbindung, Mischungen linearer Tetradecene, 1-Hexadecen, lineare Hexadecene mit innenständiger Doppelbindung und Mischungen linearer Hexadecene.

Propylen wird großtechnisch durch Spaltung von Naphtha hergestellt und ist eine Grundchemikalie, die leicht verfügbar ist. C5-Olefine, also Pentene, sind in Leichtbenzinfraktionen aus Raffinerien oder Crackern enthalten. Technische Gemische, die lineare C4-Olefine, n- und Isobutene, enthalten, sind Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden.

Die höheren Olefine können insbesondere durch Oligomerisierungsreaktionen, beispielsweise Dimerisierung, Trimerisierung oder Tetramerisierung, gewonnen werden. Geeignete Kohlenwasserstoffströme sind weiterhin das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende isomere Hexadecen (Tetrabuten) sowie durch Cooligomerisierung von Olefinen mit unterschiedlicher Kohlenstoffatomanzahl (bevorzugt 2 bis 4 C-Atome) hergestellte Olefinmischungen, gegebenenfalls nach destillativer Trennung in Fraktionen mit gleicher oder unterschiedlicher C-Zahl. Weiterhin können Olefine oder Olefinmischungen, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden.

Die bei dem Verfahren eingesetzten Olefine werden mit Synthesegas in Gegenwart eines homogen gelösten Katalysatorsystems hydroformyliert. Das molare Verhältnis zwischen Synthesegas und dem Einsatzgemisch sollte zwischen 6:1 und 1:1, vorzugsweise zwischen 3:1 und 1:1, besonders bevorzug zwischen 2:1 und 1:1 liegen. Die Hydroformylierung kann optional in Anwesenheit eines dem Fachmann bekannten Lösemittels durchgeführt wird, vorzugsweise wird jedoch kein Lösemittel verwendet.

Das bei dem erfindungsgemäßen Verfahren eingesetzte homogene Katalysatorsystem umfasst oder besteht aus Co oder Rh, vorzugsweise Rh, und vorzugsweise einem phosphorhaltigen Liganden. In einer besonders bevorzugten Ausführungsform umfasst oder besteht das erfindungsgemäße Katalysatorsystem aus Rh und einen phosphorhaltigen Liganden. Geeignete Liganden für die erfindungsgemäßen Katalysatorsysteme sind dem Fachmann bekannt (siehe z. B. die Lehrbücher "Rhodium Catalyzed Hydroformylation" (aus 2002) von P. W. N. M van Leeuwen oder "Hydrofomylation - Fundamentals, Proceses and Applications in Organic Synthesis" (aus 2016) von A. Börner und R. Franke).

Der phosphorhaltige Ligand für das erfindungsgemäße Katalysatorsystem ist vorzugsweise ein Phosphin (z. B. TPP (Triphenylphosphin)), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden.

Die Hydroformylierung wird vorzugsweise bei den folgenden Bedingungen durchgeführt: Die Temperatur bei der Hydroformylierung liegt vorzugsweise im Bereich von 80 bis 250 °C, weiterhin bevorzugt im Bereich von 90 bis 225 °C und besonders bevorzugt im Bereich von 100 bis 210 °C. Der Druck bei der Hydroformylierung liegt vorzugsweise im Bereich von 10 bis 350 bar, weiterhin bevorzugt im Bereich von 30 bis 325 bar und besonders bevorzugt im Bereich von 45 bis 300 bar.

Die Hydroformylierung wird erfindungsgemäß in mindestens einer Reaktionszone durchgeführt. Eine Reaktionszone umfasst im Sinne der vorliegenden Erfindung mindestens einen Reaktor, in dem die Hydroformylierung durchgeführt wird. Denkbar ist auch, dass die Reaktionszone mehr als einen Reaktor, insbesondere zwei oder drei Reaktoren umfasst, die parallel oder in Reihe geschaltet oder einer Mischform aus paralleler und serieller Schaltung angeordnet sein können.

Der Druck bei der Hydroformylierung entspricht üblicherweise dem Gesamtgasdruck. Der Gesamtgasdruck meint im Rahmen der vorliegenden Erfindung die Summe der vorliegenden Drücke aller vorhandenen gasförmigen Stoffe, also den Druck der (gesamten) Gasphase. Im vorliegenden Verfahren entspricht dies insbesondere der Summe der Partialdrücke von CO und H₂, d. h. der Gesamtgasdruck ist dann der Synthesegasdruck. Bei der erfindungsgemäßen Hydroformylierung macht der Partialdruckanteil von entweder CO oder H₂ maximal 70% des Gesamtgasdrucks, vorzugsweise maximal 60% des Gesamtgasdrucks, besonders bevorzugt maximal 55% des Gesamtgasdrucks aus.

Der bei der Hydroformylierung erhaltene flüssige produkthaltige Reaktionsaustrag steht demnach auch unter dem bei der Hydroformylierung vorliegenden Druck. Nach der Hydroformylierung erfolgt eine thermische Trennung oder eine Membrantrennung. Eine thermische Trennung ist hier jedoch optional, muss also nicht durchgeführt werden, wohingegen das erfindungsgemäße Verfahren immer eine Membrantrennung umfasst. Die thermische Trennung kann auch erst nach der Membrantrennung durchgeführt werden. In einem speziellen Fall kann eine Membrantrennung sowohl vor als auch nach der thermischen Trennung durchgeführt.

Die thermische Trennung, sofern ein solch optionaler Schritt durchgeführt wird, kann also vor oder nach der Membrantrennung durchgeführt werden. Die thermische Trennung kann aus einem oder mehreren Verfahrensschritten bestehen. Die thermische Trennung umfasst mindestens ein thermisches Trennverfahren, welches vorzugsweise aus Dünnschichtverdampfung, Destillation, Fallfilmverdampfung und Kurzwegverdampfung, ausgewählt wird.

In einer bevorzugten Ausführungsform der vorliegenden Erfindung erfolgt die thermische Trennung vor der Membrantrennung, der erfindungsgemäße Gasaustausch erfolgt aber nach der thermischen Trennung und vor der Membrantrennung.

Die nach der Hydroformylierung durchgeführte Membrantrennung, optional mit vorheriger oder nachfolgender thermischer Trennung wie vorgenannt beschrieben, wird zur Abtrennung des homogen gelösten Katalysatorsystems durchgeführt.

Vor der Membrantrennung, optional vor einer der Membrantrennung vorgeschalteten thermischen Trennung, wird ein Gasaustausch mit CO oder H₂ durchgeführt wird, wodurch der Partialdruckanteil anschließend von CO oder H₂ mehr als 80% des Gesamtgasdrucks, vorzugsweise mehr als 85% des Gesamtgasdrucks, besonders bevorzugt mehr als 90% des Gesamtgasdrucks ausmacht. Der Partialdruckanteil von entweder CO oder H₂ wird durch diesen Gasaustausch gezielt erhöht, während der Partialdruckanteil von der jeweils anderen Komponente verringert wird. Mögliche Verfahren zum Gasaustausch sind dem Fachmann bekannt.

Der flüssige produkthaltige Reaktionsaustrag steht auch nach der Hydroformylierung wie erwähnt unter Druck aufgrund des vorhandenen Synthesegases. Sollte vor der Membrantrennung eine thermische Trennung erfolgen, kann auch ein anderes oder ein zusätzliches Gas, beispielsweise ein Inertgas vorhanden sein. Der Gasaustausch vor der Membrantrennung erfolgt zum Beispiel dadurch, dass das vorhandene Gas zumindest teilweise abgelassen, d. h. der Reaktionsaustrag oder der Austrag aus der thermischen Trennung entspannt wird, und nachfolgend CO oder H₂ aufgedrückt wird bis der gewünschte Partialdruckanteil an CO oder H₂, also mehr als 80% des Gesamtgasdrucks, vorzugsweise mehr als 85% des Gesamtgasdrucks, besonders bevorzugt mehr als 90% des Gesamtgasdrucks, vorliegt. Eine andere Variante ist, dass nach der Entspannung des Austrags aus der Reaktion oder aus der thermischen Trennung unter Verwendung einer Mischstrecke und/oder einer Blasensäule mit CO oder H₂ beaufschlagt wird, bis der gewünschte Partialdruckanteil an CO oder H₂, also mehr als 80% des Gesamtgasdrucks, vorzugsweise mehr als 85% des Gesamtgasdrucks, besonders bevorzugt mehr als 90% des Gesamtgasdrucks, vorliegt.

Das Ablassen des Gases bzw. das Entspannen des flüssigen Reaktionsaustrags oder des Austrags aus der thermischen Trennung und das nachfolgende Aufdrücken von CO oder H₂ ggf. unter Verwendung einer Mischstrecke und/oder einer Blasensäule kann einmal oder mehrmals hintereinander durchgeführt werden, um den gewünschten Partialdruckanteil einzustellen. Nach dem Gasaustausch beträgt der Gesamtgasdruck vorzugsweise 1 bis 70 bar, weiterhin bevorzugt 2 bis 30 bar und besonders bevorzugt 4 bis 20 bar.

Nach dem Gaswechsel wird der flüssige Reaktionsaustrag oder der Austrag aus einer vorgeschalteten thermischen Trennung der Membrantrennung unterworfen, um das homogene Katalysatorsystem abzutrennen. Die Membrantrennung wird vorzugsweise bei einem Transmembrandruck von 5 bis 100 bar, weiterhin bevorzugt 10 bis 80 bar und besonders bevorzugt 20 bis 50 bar durchgeführt. Die Membrantrennung wird außerdem in einer bevorzugten Ausführungsform bei einer Temperatur von 0 °C bis 200 °C, weiterhin bevorzugt zwischen 20 °C und 160 °C durchgeführt.

Das bei der Membrantrennung eingesetzte Membranmaterial wird vorzugsweise aus der Gruppe, bestehend aus Poly(dimethyldiloxan)en, Polyimiden, partiell fluorinierten Polymeren, vollständig fluorinierten Polymeren, perfluorinierten Polymeren, amorphen Fluorpolymeren (z.B. Cytop^{®}), amorphen oder teil-kristallinen Perfluoroalkoxypolymeren (z.B. Hyflon^{®}), Block-Copolymeren der vorhergenannten Materialien, Polymeren intrinsicher Mikroporosität (PIM), Poly(aryletherketon)en, insbesondere Poly(etheretherketon)en und Poly(etherketonketon)en, Polybenzimidazolen und Polyethern, ausgewählt. Weiterhin eignen sich Polymere intrinsischer Mikroporosität (PIMs) verschiedenster Monomerzusammensetzung, insbesondere solche die Spirobifluoren-Gruppen enthalten. Das Membranmaterial ist vorzugsweise ein Poly(dimethylsiloxan) (PDMS) oder ein Perfluoroalkoxypolymer wie z.B. Hyflon^{®}.

In einer bevorzugten Ausführungsform ist das verwendete Membranmaterial im Reaktionsaustrag bzw. in dem nach der thermischen Trennung vorliegenden Gemisch intrinsisch stabil, bedarf also keiner weiteren Quervernetzung der Polymerketten mehr.

Das erfindungsgemäße Verfahren kann kontinuierlich oder diskontinuierlich durchgeführt werden. In einer bevorzugten Ausführungsform wird das Verfahren kontinuierlich durchgeführt.

Die vorliegende Erfindung wird nachfolgend anhand von Beispielen erläutert. Diese Beispiele stellen jedoch keine Beschränkung darf, sondern dienen nur der Veranschaulichung.

### Beispiele

### Beispiel 1 - Gasaustausch mit Wasserstoff und Kohlenstoffmonoxid in Toluol

### Versuchsaufbau

Dicarbonyl(acetylacetonato)rhodium(I) (1,97 g, 7,63 mmol) als Präkursor für das Übergangsmetall des Katalysatorsystems und der phosphorhaltige Ligand Alkanox^{®} 240 (Tris(2,4-di-tert-butylphenyl)phosphit)) (98,74 g, 152,6 mmol) wurden im molaren Verhältnis Rhodium : Ligand von 1 : 20 bei einer Rh-Konzentration von 300 mass-ppm in Toluol (2612,1 g) gelöst.

Mit dieser Lösung wurde eine Membrantrennung in einer Membrantestapparatur mit geschlossenem Kreislauf durchgeführt, wobei Permeat und überschüssiges Retentat wieder in den Zulauf rezykliert werden. Durch diese kontinuierliche Kreislauffahrweise wird gewährleistet, dass die Trennbedingungen für die Membran, wie z.B. Feedkonzentration und Viskosität, über den Versuchszeitraum ähnlich bleiben. Die getestete Membran mit einer Membranfläche von 84,5 cm² wurde in einer Flachkanaltestzelle gedichtet. Als Membranmaterial wurde quervernetztes Poly(dimethylsiloxan) (PDMS) verwendet.

Zunächst wurde der Katalysator bei Synthesegasdruck von 40 bar bei 60 °C im Reaktor ohne Membrantrennung präformiert. Danach wurde der Reaktor auf 20 bar Gesamtgasdruck entspannt, die Temperatur auf 30 °C gesenkt und anschließend die Flachkanaltestzellen in Betrieb genommen. Der Membrankreislauf wurde bei 30 °C sowie 50 bar Feeddruck und 21 bar Permeatvordruck betrieben.

### Messung des Rückhaltes

Die erste Messung, Messung 1, erfolgte bei einem Reaktordruck von 20 bar Synthesegas (Mischung CO : H₂ = ca. 50 : 50). Von Feed, Retentat und Permeat wurden jeweils Proben genommen und auf ihre Rhodium-Konzentration mittels ICP-MS hin untersucht, um daraus den Rhodium-Rückhalt zu ermitteln (Rhodium-Rückhalt = 1 - (Konzentration Rh im Permeat / Konzentration Rh im Retentat). Der Rhodium-Rückhalt belief sich auf 73%.

Bei laufender Membrantrennung wurde anschließend ein Gaswechsel durchgeführt, in dem der Reaktor mehrmals auf 2 bar entspannt wurde und nachfolgend wieder mit Wasserstoff auf 20 bar aufgedrückt wurde. Dieser Druckwechsel bestehend aus Entspannen und Aufdrücken mit Wasserstoff wurde dreimal wiederholt. Der Partialdruckanteil von Wasserstoff hat danach mehr als 90% des Gesamtgasdrucks ausgemacht. Anschließend wurde Messung 2 mit diesem Feed durchgeführt. Sowohl von Feed als auch von Permeat wurden Proben genommen und auf ihre Rhodium-Konzentration untersucht, um daraus den Rhodium-Rückhalt zu ermitteln. Der Rhodium-Rückhalt belief sich nach dem Aufdrücken von Wasserstoff auf 80%. Es zeigt sich, dass der Rückhalt von Rhodium bei Membrantrennung durch die vorherige Beaufschlagung mit Wasserstoff statt Synthesegas deutlich erhöht werden konnte.

Anschließend wurde ähnlich des Gaswechsels von Synthesegas auf Wasserstoff der umgekehrte Gaswechsel wieder von Wasserstoff auf Synthesegasdruck durchgeführt, um den Ausgangspunkt wiederherzustellen (Messung 3). Der Rhodium-Rückhalt belief sich hiernach auf 70%.

Hiernach wurde in ähnlicher Weise erneut ein Gaswechsel durchgeführt, wobei diesmal der Gaswechsel auf Kohlenstoffmonoxid durchgeführt wurde (Messung 4). Der Rhodium-Rückhalt belief sich hiernach auf 78 %. Es zeigt sich, dass der Rückhalt von Rhodium bei Membrantrennung auch durch die vorherige Beaufschlagung mit Kohlenstoffmonoxid statt Synthesegas deutlich erhöht werden konnte.

Die Ergebnisse sind nachfolgend nochmal tabellarisch zusammengefasst:

| Messung | Membranrückhalt Rhodium |
|---|---|
| 1 (Synthesegas) | 73 % |
| 2 (Wasserstoff) | 80 % |
| 3 (Synthesegas) | 70 % |
| 4 (Kohlenstoffmonoxid) | 78 % |

### Beispiel 2 - Gasaustausch mit Wasserstoff und Kohlenmonoxid in Isotridecanal

Der in Beispiel 1 beschriebene Versuch wurde mit Isotridecanal statt Toluol durchgeführt. Hierzu wurden Dicarbonyl(acetylacetonato)rhodium(I) (1,174 g, 4,55 mmol) und Alkanox^{®} 240 (Tris(2,4-di-tert-butylphenyl)phosphit)) (58,31 g, 152,6 mmol) im molaren Verhältnis Rhodium : Ligand von 1 : 20 bei einer Rh-Konzentration von 300 mass-ppm in Isotridecanal (2492,02 g) gelöst. Der Versuchsaufbau und die Durchführung wurden wie in Beispiel 1 beschrieben durchgeführt.

Es zeigte sich auch hier, dass sowohl nach Beaufschlagung mit Wasserstoff als auch noch Beaufschlagung mit Kohlenstoffmonoxid ein höherer Membranrückhalt vorlag als im Vergleichsintervall mit Synthesegas.

Die Ergebnisse mit Isotridecanal als Lösemittel sind nachfolgend tabellarisch zusammengefasst:

| Messung | Membranrückhalt Rhodium |
|---|---|
| 1 (Synthesegas) | 30 % |
| 2 (Wasserstoff) | 45 % |
| 3 (Synthesegas) | 21 % |
| 4 (Kohlenstoffmonoxid) | 40 % |

### Beispiel 3 - Gasaustausch mit Wasserstoff und Kohlenmonoxid in Toluol mit Membran aus Poly(etheretherketon)

Der in Beispiel 1 beschriebene Versuch wurde mit Toluol und mit einer Membran aus Poly(etheretherketon) (PEEK) statt mit einer Membran aus quervernetztem PDMS durchgeführt. Die Membran aus PEEK wurde nach WO 2015/110843 A1 (Beispiel 1) hergestellt.

Es wurden Dicarbonyl(acetylacetonato)rhodium(I) (1,824 g, 7,07 mmol) und Alkanox^{®} 240 (Tris(2,4-di-tert-butylphenyl)phosphit)) (90,85 g, 140,4 mmol) im molaren Verhältnis Rhodium : Ligand von 1 : 20 bei einer Rh-Konzentration von 300 mass-ppm in Toluol (2431 g) gelöst. Der Versuchsaufbau und die Durchführung wurden wie in Beispiel 1 beschrieben durchgeführt.

Es zeigte sich auch hier, dass sowohl nach Beaufschlagung mit Wasserstoff als auch noch Beaufschlagung mit Kohlenstoffmonoxid ein höherer Membranrückhalt vorlag als im Vergleichsintervall mit Synthesegas.

Die Ergebnisse mit Toluol als Lösemittel und einer Membran aus PEEK sind nachfolgend tabellarisch zusammengefasst:

| Messung | Membranrückhalt Rhodium |
|---|---|
| 1 (Synthesegas) | 89 % |
| 2 (Wasserstoff) | 96 % |
| 3 (Synthesegas) | 90 % |
| 4 (Kohlenstoffmonoxid) | 95 % |

### Beispiel 4 - Gasaustausch mit Wasserstoff und Kohlenmonoxid in Toluol mit SolSep NF030306

Der in Beispiel 1 beschriebene Versuch wurde mit einer kommerziell verfügbaren Membran, NF030306 von SolSep BV, durchgeführt.

Es wurden Dicarbonyl(acetylacetonato)rhodium(I) (1,825 g, 7,07 mmol) und Alkanox^{®} 240 (Tris(2,4-di-tert-butylphenyl)phosphit)) (90,90 g, 140,5 mmol) im molaren Verhältnis Rhodium : Ligand von 1 : 20 bei einer Rh-Konzentration von 300 mass-ppm in Toluol (2448 g) gelöst. Der Versuchsaufbau und die Durchführung wurden wie in Beispiel 1 beschrieben durchgeführt.

Es zeigte sich auch hier, dass sowohl nach Beaufschlagung mit Wasserstoff als auch noch Beaufschlagung mit Kohlenstoffmonoxid ein höherer Membranrückhalt vorlag als im Vergleichsintervall mit Synthesegas.

Die Ergebnisse mit Toluol als Lösemittel und der Membran SolSep NF030306 sind nachfolgend tabellarisch zusammengefasst:

| Messung | Membranrückhalt Rhodium |
|---|---|
| 1 (Synthesegas) | 63 % |
| 2 (Wasserstoff) | 74 % |
| 3 (Synthesegas) | 66 % |
| 4 (Kohlenstoffmonoxid) | 71 % |

## Patentansprüche

1. Verfahren zur Herstellung von Aldehyden, wobei das Verfahren zumindest
eine Hydroformylierung durch Umsetzung von C2- bis C20-Olefinen, vorzugsweise C2- bis C17-Olefinen, weiterhin bevorzugt C3 bis C14-Olefinen und ganz besonders bevorzugt C6-bis C14-Olefinen, mit Synthesegas in Gegenwart eines homogen gelösten Katalysatorsystems, welches mindestens Co oder Rh und vorzugsweise einen phosphorhaltigen Liganden umfasst, in mindestens einer Reaktionszone unter Erhalt eines flüssigen produkthaltigen Reaktionsaustrags, wobei bei der Hydroformylierung der Partialdruckanteil von CO oder H₂ maximal 75% des Gesamtgasdrucks, vorzugsweise maximal 70% des Gesamtgasdrucks, besonders bevorzugt maximal 65% des Gesamtgasdrucks ausmacht, wobei der Gesamtgasdruck die Summe der vorliegenden Drücke aller vorhandenen gasförmigen Stoffe ist, und
eine Membrantrennung zur Abtrennung des homogen gelösten Katalysatorsystems umfasst, **dadurch gekennzeichnet, dass**
vor der Membrantrennung ein Gasaustausch mit CO oder H₂ durchgeführt wird, wodurch der Partialdruckanteil von CO oder H₂ mehr als 80% des Gesamtgasdrucks, vorzugsweise mehr als 85% des Gesamtgasdrucks, besonders bevorzugt mehr als 90% des Gesamtgasdrucks ausmacht.

2. Verfahren nach Anspruch 1, wobei die Hydroformylierung bei einem Druck von 10 bis 350 bar durchgeführt wird.

3. Verfahren nach Anspruch 1 oder 2, wobei die Hydroformylierung bei einer Temperatur von 80 bis 250 °C durchgeführt wird.

4. Verfahren nach einem der Ansprüche 1 bis 3, wobei das homogen gelöste Katalysatorsystems mindestens Rh und einen phosphorhaltigen Liganden umfasst.

5. Verfahren nach einem der Ansprüche 1 bis 4, wobei der phosphorhaltige Ligand ein Phosphin, ein Monophosphit, ein Bisphosphit oder eine Mischungen daraus ist.

6. Verfahren nach einem der Ansprüche 1 bis 5, wobei der Gesamtgasdruck nach dem Gasaustausch 1 bis 70 bar, vorzugsweise 2 bis 30 bar, vorzugsweise 4 bis 20 bar beträgt.

7. Verfahren nach einem der Ansprüche 1 bis 6, wobei die Membrantrennung bei einem Transmembrandruck von 5 bis 100 bar, vorzugsweise 10 bis 80 bar, besonders bevorzugt 20 bis 50 bar durchgeführt wird.

8. Verfahren nach einem der Ansprüche 1 bis 7, wobei die Membrantrennung bei einer Temperatur von 0 °C bis 200 °C, bevorzugt zwischen 20 °C und 160 °C durchgeführt wird.

9. Verfahren nach einem der Ansprüche 1 bis 8, wobei vor oder nach der Membrantrennung mindestens eine thermische Trennung durchgeführt wird.

10. Verfahren nach Anspruch 9, wobei die thermische Trennung mindestens ein thermisches Trennverfahren umfasst, welches aus Dünnschichtverdampfung, Destillation, Fallfilmverdampfung und Kurzwegverdampfung, ausgewählt wird.

11. Verfahren nach Anspruch 9 oder 10, wobei die thermische Trennung vor der Membrantrennung, der Gasaustausch aber nach der thermischen Trennung und vor der Membrantrennung erfolgt.

12. Verfahren nach einem der Ansprüche 1 bis 11, wobei das Verfahren kontinuierlich durchgeführt wird.

13. Verfahren nach einem der Ansprüche 1 bis 12, wobei die Reaktionszone mindestens einen Reaktor umfasst.

14. Verfahren nach einem der Ansprüche 1 bis 13, wobei bei der Membrantrennung ein Membranmaterial, ausgewählt aus der Gruppe, bestehend aus Poly(dimethyldiloxan)en, Polyimiden, partiell fluorinierten Polymeren, vollständig fluorinierten Polymeren, perfluorinierten Polymeren, amorphen Fluorpolymeren (z.B. Cytop), amorphen oder teil-kristallinen Perfluoroalkoxypolymeren (z.B. Hyflon), Block-Copolymeren der vorhergenannten Materialien, Polymeren intrinsicher Mikroporosität (PIM), Poly(aryletherketon)en, insbesondere Poly(etheretherketon)en und Poly(etherketonketon)en, Polybenzimidazolen und Polyethern, eingesetzt wird.

15. Verfahren nach Anspruch 14, wobei ein Membranmaterial verwendet wird, welches im Reaktionsaustrag bzw. in dem nach der thermischen Trennung vorliegenden Gemisch intrinsisch stabil ist, also keiner weiteren Quervernetzung der Polymerketten mehr bedarf.

## Claims

1. Process for preparing aldehydes, said process comprising at least
a hydroformylation through the reaction of C2 to C20 olefins, preferably C2 to C17 olefins, more preferably C3 to C14 olefins and most preferably C6 to C14 olefins, with syngas in the presence of a homogeneously dissolved catalyst system that comprises at least Co or Rh and preferably a phosphorus-containing ligand, in at least one reaction zone, yielding a liquid reaction output containing the product, wherein the partial pressure fraction of CO or H₂ in the hydroformylation constitutes not more than 75% of the total gas pressure, preferably not more than 70% of the total gas pressure, more preferably not more than 65% of the total gas pressure, the total gas pressure being the sum of the pressures occurring from all the gaseous substances present, and
a membrane separation for separating the homogeneously dissolved catalyst system, **characterized in that**,
before the membrane separation, a gas exchange with CO or H₂ is carried out, as a result of which the partial pressure fraction of CO or H₂ constitutes more than 80% of the total gas pressure, preferably more than 85% of the total gas pressure, more preferably more than 90% of the total gas pressure.

2. Process according to Claim 1, wherein the hydroformylation is carried out at a pressure of 10 to 350 bar.

3. Process according to Claim 1 or 2, wherein the hydroformylation is carried out at a temperature of 80 to 250°C.

4. Process according to any of Claims 1 to 3, wherein the homogeneously dissolved catalyst system comprises at least Rh and a phosphorus-containing ligand.

5. Process according to any of Claims 1 to 4, wherein the phosphorus-containing ligand is a phosphine, a monophosphite, a bisphosphite or mixtures thereof.

6. Process according to any of Claims 1 to 5, wherein the total gas pressure after the gas exchange is 1 to 70 bar, preferably 2 to 30 bar, preferably 4 to 20 bar.

7. Process according to any of Claims 1 to 6, wherein the membrane separation is carried out at a transmembrane pressure of 5 to 100 bar, preferably 10 to 80 bar, more preferably 20 to 50 bar.

8. Process according to any of Claims 1 to 7, wherein the membrane separation is carried out at a temperature of 0 to 200°C, preferably between 20°C and 160°C.

9. Process according to any of Claims 1 to 8, wherein at least one thermal separation is carried out before or after the membrane separation.

10. Process according to Claim 9, wherein the thermal separation comprises at least one thermal separation process selected from thin-film evaporation, distillation, falling-film evaporation and short-path evaporation.

11. Process according to Claim 9 or 10, wherein the thermal separation takes place before the membrane separation, but the gas exchange takes place after the thermal separation and before the membrane separation.

12. Process according to any of Claims 1 to 11, wherein the process is carried out continuously.

13. Process according to any of Claims 1 to 12, wherein the reaction zone comprises at least one reactor.

14. Process according to any of Claims 1 to 13, wherein the membrane separation uses a membrane material selected from the group consisting of polydimethylsiloxanes, polyimides, partially fluorinated polymers, completely fluorinated polymers, perfluorinated polymers, amorphous fluoropolymers (e.g. Cytop), amorphous or partially crystalline perfluoroalkoxy polymers (e.g. Hyflon), block copolymers of the abovementioned materials, polymers having intrinsic microporosity (PIM), polyaryletherketones, in particular polyetheretherketones and polyetherketoneketones, polybenzimidazoles and polyethers.

15. Process according to Claim 14, wherein a membrane material is used that is intrinsically stable in the reaction output/in the mixture present after the thermal separation, i.e. it does not require any additional crosslinking of the polymer chains.

## Revendications

1. Procédé destiné à la préparation d'aldéhydes, le procédé comprenant au moins
une hydroformylation par réaction d'oléfines en C2-C20, de préférence d'oléfines en C2-C17, plus préférablement d'oléfines en C3-C14 et tout particulièrement préférablement d'oléfines en C6-C14, avec un gaz de synthèse en présence d'un système catalytique dissous de manière homogène, qui comprend au moins Co ou Rh et de préférence un ligand contenant du phosphore, dans au moins une zone de réaction pour obtenir un effluent de réaction liquide contenant un produit, lors de l'hydroformylation, la proportion de pression partielle de CO ou H₂ représentant au maximum 75 % de la pression totale de gaz, de préférence au maximum 70 % de la pression totale de gaz, particulièrement préférablement au maximum 65 % de la pression totale de gaz, la pression totale de gaz étant la somme des pressions existantes de toutes les substances gazeuses présentes, et
une séparation membranaire pour séparer le système catalytique dissous de manière homogène, **caractérisé en ce que**,
avant la séparation membranaire, un échange de gaz avec CO ou H₂ est effectué, ce par quoi la proportion de pression partielle de CO ou de H₂ représente plus de 80 % de la pression totale de gaz, de préférence plus de 85 % de la pression totale de gaz, particulièrement préférablement plus de 90 % de la pression totale de gaz.

2. Procédé selon la revendication 1, dans lequel l'hydroformylation est effectuée à une pression de 10 à 350 bar.

3. Procédé selon la revendication 1 ou 2, dans lequel l'hydroformylation est effectuée à une température de 80 à 250 °C.

4. Procédé selon l'une quelconque des revendications 1 à 3, dans lequel le système catalytique dissous de manière homogène comprend au moins Rh et un ligand contenant du phosphore.

5. Procédé selon l'une quelconque des revendications 1 à 4, dans lequel le ligand contenant du phosphore est une phosphine, un monophosphite, un biphosphite ou un mélange de ceux-ci.

6. Procédé selon l'une quelconque des revendications 1 à 5, dans lequel la pression totale de gaz après l'échange de gaz est de 1 à 70 bar, de préférence de 2 à 30 bar, de préférence de 4 à 20 bar.

7. Procédé selon l'une quelconque des revendications 1 à 6, dans lequel la séparation membranaire est effectuée à une pression transmembranaire de 5 à 100 bar, de préférence de 10 à 80 bar, particulièrement préférablement de 20 à 50 bar.

8. Procédé selon l'une quelconque des revendications 1 à 7, dans lequel la séparation membranaire est effectuée à une température de 0 °C à 200 °C, de préférence entre 20 °C et 160 °C.

9. Procédé selon l'une quelconque des revendications 1 à 8, dans lequel au moins une séparation thermique est effectuée avant ou après la séparation membranaire.

10. Procédé selon la revendication 9, dans lequel la séparation thermique comprend au moins un procédé de séparation thermique, qui est choisi parmi l'évaporation en couche mince, la distillation, l'évaporation à film tombant et l'évaporation à court trajet.

11. Procédé selon la revendication 9 ou 10, dans lequel la séparation thermique a lieu avant la séparation membranaire, mais l'échange de gaz a lieu après la séparation thermique et avant la séparation membranaire.

12. Procédé selon l'une quelconque des revendications 1 à 11, le procédé étant effectué en continu.

13. Procédé selon l'une quelconque des revendications 1 à 12, dans lequel la zone de réaction comprend au moins un réacteur.

14. Procédé selon l'une quelconque des revendications 1 à 13, dans lequel est utilisée, lors de la séparation membranaire, une matière de membrane choisie dans le groupe constitué par des poly(diméthyldiloxanes), des polyimides, des polymères partiellement fluorés, des polymères entièrement fluorés, des polymères perfluorés, des polymères fluorés amorphes (par exemple Cytop), des polymères perfluoroalcoxy amorphes ou partiellement cristallins (par exemple Hyflon), des copolymères à blocs des matières susmentionnées, des polymères à microporosité intrinsèque (PIM), des poly(aryléthercétones), en particulier des poly(étheréthercétones) et des poly(éthercétonecétones), des polybenzimidazoles et des polyéthers.

15. Procédé selon la revendication 14, dans lequel une matière de membrane est utilisée qui est intrinsèquement stable dans l'effluent de réaction ou dans le mélange présent après la séparation thermique, c'est-à-dire qui ne nécessite aucune réticulation supplémentaire des chaînes polymères.
